# EUROPEAN PATENT SPECIFICATION

(11) **EP 4 556 061 B1**
(45) Date of publication and mention of the grant of the patent: **22.04.2026**
(21) Application number: 23210911.6
(22) Date of filing: 20.11.2023
(51) Int. Cl.: A61N 1/39, A61B 5/29, A61B 5/361, A61B 5/363, A61B 5/00, A61N 1/36, A61N 1/362, A61N 1/365

(54) **METHOD FOR PROCESSING AN OUTPUT SIGNAL FROM A SENSOR OF AN IMPLANTABLE MEDICAL DEVICE**
VERFAHREN ZUR VERARBEITUNG EINES AUSGANGSSIGNALS AUS EINEM SENSOR EINER IMPLANTIERBAREN MEDIZINISCHEN VORRICHTUNG
PROCÉDÉ DE TRAITEMENT D'UN SIGNAL DE SORTIE PROVENANT D'UN CAPTEUR D'UN DISPOSITIF MÉDICAL IMPLANTABLE

(43) Date of publication of application: 21.05.2025
(73) Proprietor: BIOTRONIK SE & Co. KG, 12359 Berlin (DE)
(72) Inventor: Weiss, Ingo, 12435 Berlin (DE); Dörr, Thomas, 12437 Berlin (DE)
(74) Representative: Biotronik Corporate Services SE

(56) References cited:
- US-A1- 2005 149 134
- US-B2- 11 617 534
- US-B2- 8 068 901

## Description

The present invention relates to a computer-implemented method for processing an output signal from a sensor of an implantable medical device, in particular an implantable cardioverter-defibrillator. The invention further relates to a controller, a computer program and a computer-readable medium for carrying out this method, as well as to an implantable medical device.

An implantable medical device, such as an implantable cardioverter-defibrillator (ICD) or a pacemaker, may be equipped with a filter configured to remove or reduce interference in an output signal from a heart sensor of the device. Common filters may include high-pass, lowpass, band-pass and notch filters. In some cases, interference in the form of short, quasi-periodically recurring pulses may occur in the output signal, which may be misinterpreted by a controller of the device, e.g. as normal heart activity. However, using the above filters to remove or reduce such interference may overly affect the useful components of the output signal.

US11617534 discloses a medical device and a method for detecting electrical signal noise.

It may therefore be considered an objective of the present invention to provide an improved method for processing an output signal from a sensor of an implantable medical device. More specifically, the objective may be to provide a method for processing the output signal in such a manner that pulse-like interference patterns in the output signal are removed or reduced without overly affecting its useful components. Another objective of the invention may be to provide a controller, a computer program and a computer-readable medium for carrying out this method, as well as an improved implantable medical device, in particular an improved implantable cardioverter-defibrillator.

These objectives may be achieved by the subject-matter of the independent claims. Advantageous embodiments are defined in the dependent claims as well as in the corresponding specification and figures.

A first aspect of the invention relates to a computer-implemented method for processing an output signal from a sensor of an implantable medical device, in particular an implantable cardioverter-defibrillator, the sensor configured to generate the output signal from an electrical heart signal from a patient's heart. The method comprises at least the following steps: receiving the output signal; identifying an undesired pulse pattern in the output signal; determining a first portion of the output signal, the first portion including the undesired pulse pattern; modifying the output signal by replacing the first portion with a predetermined second portion that lacks the undesired pulse pattern to obtain a modified output signal.

The method makes it possible to eliminate, i.e. reduce or remove, impulse-like disturbances (which could be misinterpreted as a physiological heart signal) in order to make an underlying actual physiological heart signal more recognisable.

This minimises the risk of misinterpretation by the controller of the implantable medical device. In particular, the method may help to make detection of ventricular fibrillation and/or ventricular tachycardia with an ICD more robust against pulse artefacts from co-existing medical devices, such as pacemakers, in particular leadless pacemakers, pain management implants, brain stimulation implants, cardiac contractility modulation (CCM) devices, functional electrical stimulation (FES) devices, hyperthermia or MRI systems.

The method may be carried out automatically by a processor.

The output signal may be a digital and/or analogue signal.

Generally, the undesired pulse pattern in the output signal may result from interference between the heart signal and one or more foreign electrical signals induced in the patient's body by one or more external devices (which may be implanted in the patient's body). Such interference may affect the useful components of the output signal.

For example, the undesired pulse pattern may be characterised by one or more (Dirac-like) impulses whose amplitude is significantly higher (e.g. at least 10, 20, 50, 100, 1,000, 10,000 percent higher) than an average amplitude of the output signal.

The undesired pulse pattern may be identified by analysing the output signal with a set of parameters defining one or more characteristics of the undesired pulse pattern, such as a frequency band, an amplitude (e.g. in specific frequency bands), a degree of periodicity, a pulse width (above a specified amplitude threshold), an edge steepness, a number of (local) extrema, a number of inflection points.

The parameters may describe one or more characteristics of the undesired pulse pattern in the time and/or frequency domain.

The set of parameters may be stored in a look-up table (which may be stored in a memory of the implantable medical device). Alternatively, one or more mathematical functions (which may be stored in the memory of the implantable medical device) may be used to identify the undesired pulse pattern.

It is possible that different variants of the undesired pulse pattern are identified in the output signal. In this case, the first portion may include one or at least two of these variants.

To identify the undesired pulse pattern, one or more characteristics of the output signal may be compared with expected values, and the closeness of the match may be used to quantify the certainty with which the undesired pulse pattern was found.

In particular, the undesired pulse pattern may be identified depending on a degree of correlation of the undesired pulse pattern to a reference pattern, e.g. by comparing a correlation coefficient determined from both patterns to a threshold, or by comparing an absolute maximum determined from a convolution of the undesired pulse pattern with a time-mirrored reference pattern to a threshold.

The second portion, with which the first portion of the output signal from the sensor is to be replaced, may include a (predetermined) desired pulse pattern different from the undesired pulse pattern. The second portion may include one variant or different variants of the desired pulse pattern.

The first portion and the second portion may have equal or different lengths (i.e. durations). In particular, the second portion may be shorter than the first portion. In some cases, the second portion may even have a length of zero. In the latter case, further evaluation of the modified output signal (e.g. by a control unit for controlling a pulse generator of the implantable medical device) may pause for the time that the second portion is too short in order to stay in sync with the original signal.

However, it is also possible that the second portion is longer than the first portion and is inserted into the output signal so as to overlap the output signal in a portion (immediately) preceding and/or (immediately) following the first portion. This may avoid undesired irregularities in the output signal when inserting the second portion into the output signal. The resulting transitions may additionally be smoothed out, e.g. using a (linear) fading function.

The first portion and the undesired pulse pattern may have equal or different lengths. In the latter case, the undesired pulse pattern may be offset by a predetermined time to a beginning and/or end of the first portion (see below).

A second aspect of the invention relates to a controller for an implantable medical device, in particular an implantable cardioverter-defibrillator. The controller comprises a processor configured to carry out the method as described above and below. In addition to the processor, the controller may comprise a memory and one or more data communication interfaces for data communication with peripheral devices, e.g. a remote, a smartphone, a smartwatch, a tablet, a laptop, a PC, a server. The controller may comprise hardware and/or software modules.

A third aspect of the invention relates to an implantable medical device, in particular an implantable cardioverter-defibrillator, comprising a sensor configured to generate an output signal from an electrical heart signal from a patient's heart and the controller as described above and below. The sensor may be implantable in the patient's body. The sensor may comprise one or more electrodes that are in direct contact with one or more portions of the patient's body when the sensor is implanted. For example, the electrode(s) may be part of an implantable lead and/or of an implantable housing of the implantable medical device.

The implantable medical device may be, for example, an implantable pulse generator (IPG), an (extravascular, i.e. subcutaneous, or transvenous) implantable cardioverter-defibrillator (ICD), a cardiac resynchronization therapy (CRT) pacemaker, a neurostimulator, an electrocardiogram (ECG) recorder.

The implantable medical device may comprise a signal processing unit configured to modify the output signal with the method as described above and below.

Generally, the signal processing unit may be configured to filter and/or amplify the output signal before further processing by the implantable medical device.

The implantable medical device may additionally comprise a control unit configured to generate, from an input signal, a control signal for controlling a pulse generator of the implantable medical device. The pulse generator may be configured to generate, from the control signal, an electrical pulse for electrically stimulating the patient's heart. In this case, the signal processing unit may be configured to provide the modified output signal and/or the (unmodified, or filtered and/or amplified) output signal as the input signal.

At least one or all of the above units may be implemented as hardware and/or software modules of the controller.

Further aspects of the invention relate to a computer program comprising instructions which, when the program is executed by a processor, cause the processor to carry out the method as described above and below, as well as to a computer-readable medium in which the computer program is stored. The computer program may be executed by a processor of the controller.

The computer-readable medium may be a volatile or non-volatile data storage device. For example, the computer-readable medium may be a hard drive, a USB (universal serial bus) storage device, a RAM (random-access memory), a ROM (read-only memory), an EPROM (erasable programmable read-only memory), a flash memory or a combination of at least two of these examples. The computer-readable medium may also be a data communication network for downloading program code, such as the Internet or a data cloud.

It should be noted that features of the method as described above and below may be features of the controller, the computer program and the computer-readable medium, and vice versa.

Embodiments of the invention may be considered, without limiting the invention, as being based on the ideas and findings described below.

According to an embodiment, the output signal may have been generated by the sensor during abnormal activity of the patient's heart, e.g. during ventricular fibrillation or ventricular tachycardia. Additionally or alternatively, the undesired pulse pattern may be similar to a characteristic pulse pattern occurring in the output signal (and/or the heart signal) during normal activity of the patient's heart and/or during pacing of the patient's heart with a pacemaker (which may be external and/or not operably coupled to the implantable medical device). For example, the sensor may have generated the output signal by sensing interference between the (physiological) heart signal, in particular the abnormal heart signal during abnormal activity of the patient's heart (e.g. ventricular fibrillation or ventricular tachycardia), and an electrical stimulation signal that electrically stimulates at least a part of the patient's body (and is generated by a stimulation device that is external and/or not operably coupled to the implantable medical device), in particular the pacing signal. As a result, the characteristic pulse pattern may be present as the undesired pulse pattern in the output signal. For example, the undesired pulse pattern may be similar to a P wave, a (part of a) QRS complex, a T wave, a U wave or a combination of at least two of these examples.

According to an embodiment, the method may further comprise determining a third portion of the output signal, the third portion being outside the undesired pulse pattern; determining a desired pulse pattern from the third portion; generating the second portion, the second portion including the desired pulse pattern.

The desired pulse pattern may be similar to an actual pulse pattern of the output signal outside the identified undesired pulse pattern(s). However, the desired pulse pattern may also significantly differ from the actual pulse pattern. In some cases, the desired pulse pattern may be a flat line or have a length (i.e. duration) of zero (see above).

The desired pulse pattern may be determined according to a set of parameters defining one or more characteristics of the desired pulse pattern, such as a frequency band, an amplitude (e.g. in specific frequency bands), a degree of periodicity, a pulse width (above a specified amplitude threshold), an edge steepness, a number of (local) extrema, a number of inflection points.

The set of parameters may be stored in a look-up table (which may be stored in a memory of the implantable medical device). Alternatively, one or more mathematical functions (which may be stored in the memory of the implantable medical device) may be used to determine the desired pulse pattern.

It is possible that different variants of the desired pulse pattern are determined from the third portion. In this case, the second portion may include one or at least two of these variants.

In some cases, the desired pulse pattern may be too short. Further evaluation of the modified output signal may then be suspended during the resulting gaps. Alternatively, the gaps may be closed by interpolation, especially by linear interpolation.

It is also possible that the controller, when identifying the undesired pulse pattern, may act as if the desired pulse pattern had been evaluated (instead of inserting the second portion into the output signal).

The second portion may begin a predetermined time before a beginning of the desired pulse pattern and/or end a predetermined time after an end of the desired pulse pattern. For example, the predetermined time may be determined depending on a length of the first portion and/or a length of the desired pulse pattern. The predetermined time may be positive or negative. For example, the predetermined time may be 1 min or less, 30 s or less, 10 s or less, 5 s or less, 1 s or less, 0.1 s or less, or 0. The predetermined time may be constant or may be varied when the method is carried out.

Alternatively, the second portion and the desired pulse pattern may have the same length.

The third portion may end before a beginning of the undesired pulse pattern and/or begin after an end of the undesired pulse pattern. In particular, the third portion may immediately precede and/or follow the undesired pulse pattern. In other words, the third portion may be adjacent to the beginning and/or end of the undesired pulse pattern.

The third portion may additionally be outside the first portion.

According to an embodiment, determining the desired pulse pattern may comprise searching for characteristic peaks in a spectrum of the third portion and determining the desired pulse pattern according to frequencies associated with the found characteristic peaks. The characteristic peaks may be peaks above a specified amplitude threshold and/or peaks in one or more specified frequency bands.

Additionally or alternatively, determining the desired pulse pattern may comprise determining a degree of periodicity from the third portion and determining the desired pulse pattern according to the degree of periodicity. For example, the degree of periodicity may be determined by applying an autocorrelation function to the third portion. The degree of periodicity may be used to generate a synthetic signal with the desired pulse pattern, e.g. a sine wave with a period (and amplitude) similar to a period (and amplitude) of the third portion. Alternatively, the third portion may be repeated in a length less than or equal to a determined period in rhythm with that period.

Additionally or alternatively, at least a part of the desired pulse pattern may be a copy of at least a part of the third portion.

This ensures that the second portion in the modified output signal is similar or even identical to an unmodified portion of the modified output signal.

According to an embodiment, the first portion may begin a predetermined time before a beginning of the undesired pulse pattern and/or end a predetermined time after an end of the undesired pulse pattern.

For example, the predetermined time may be determined depending on a length of the second portion and/or a length of the undesired pulse pattern. Additionally or alternatively, the predetermined time may be determined depending on one or more characteristics determined from a third portion of the output signal, the third portion being outside the undesired pulse pattern. The predetermined time may be positive or negative. For example, the predetermined time may be 1 min or less, 30 s or less, 10 s or less, 5 s or less, 1 s or less, 0.1 s or less, or 0. The predetermined time may be constant or may be varied when the method is carried out.

This has the advantage that potentially undesired portions of the output signal (immediately) preceding and/or (immediately) following the identified undesired pulse pattern can also be eliminated by replacing the first portion with the second portion.

Alternatively, the first portion and the undesired pulse pattern may have the same length.

According to an embodiment, a current repetition of the undesired pulse pattern may be identified considering at least one previously identified repetition of the undesired pulse pattern, in particular considering a time interval between at least two previously identified repetitions. This may improve the accuracy of the method.

The undesired pulse pattern may be considered repeated when, after a first repetition at a first time interval, it is repeated at least a second time at a second time interval, the second time interval differing from the first time interval by less than a predetermined tolerance, and/or when a first repetition and a second repetition of the undesired pulse pattern differ from each other (e.g. in at least one of the above parameters) by less than a predetermined threshold.

According to an embodiment, a time frame in which the current repetition is likely to occur may be estimated from a time interval between at least two previously identified repetitions. The undesired pulse pattern may then be identified in the time frame.

The time interval may have been measured and/or calculated. For example, the time interval may be an average calculated from at least two measured time intervals, each of which measured between two previously identified (consecutive) repetitions.

In particular, each previously identified repetition used to determine the time interval may have been identified with an actual accuracy that is above a required accuracy.

The time interval may be updated continuously.

This may further improve the accuracy of the method.

According to an embodiment, the current repetition may not be identified in the time frame when an actual accuracy with which the undesired pulse pattern has been last identified is below a required accuracy. Additionally or alternatively, (at least a part of) the output signal in the time frame may be used as the first portion when the current repetition is not identified in the time frame. In other words, it may not be absolutely necessary to search for the current repetition in the time frame in order to be able to generate the modified output signal. Instead, the modified output signal may be generated by directly inserting the second portion into the time frame. This has the advantage that the undesired pulse pattern can be suppressed even if the accuracy is temporarily reduced for some reason. So, the quality of the filter effect caused by the method can be kept constant.

This prognostic continuation may be terminated after a specified duration and/or when the actual accuracy corresponds again to the required accuracy.

According to an embodiment, the undesired pulse pattern may be identified by comparing an amplitude of the output signal with at least one threshold value. For example, the threshold value(s) may be used to determine a maximum, minimum or average value of the amplitude, or a combination of at least two of these values, within a specified period of time. This may simplify the implementation of the method in software and/or hardware. This may also improve the efficiency of the method.

It is possible that different threshold values are used to identify one current repetition of the undesired pulse pattern.

According to an embodiment, an amplitude of each previously identified repetition may be determined. In this case, the at least one threshold value may be determined from the amplitude of at least one previously identified repetition and/or from a trend determined from the amplitude of at least two previously identified repetitions. In other words, the threshold value(s) may be continuously adjusted to a change in the amplitude of the undesired pulse pattern, e.g. by increasing it (them) when the amplitude increases in magnitude or decreasing it (them) when the amplitude decreases in magnitude. For example, the amplitude of each previously identified repetition may be a maximum, minimum or average value, or a combination of at least two of these values, determined over the duration of that repetition. The threshold value(s) may then be calculated from one or more of these values. This may further improve the accuracy of the method.

For example, extrema in the amplitude of the output signal beyond the threshold value(s) may be tracked, and a trend may be determined from the tracked extrema. A forecast for a later adjustment of the threshold value(s) may then be derived from the trend. The forecast may be realised by linear or exponential (in particular exponentially decaying) continuation of the previous trend. Polynomials of higher order (generally realised as Taylor development) may be used as well.

According to an embodiment, the method may further comprise generating a control signal from the modified output signal, the control signal causing a pulse generator of the implantable medical device to generate an electrical pulse for electrically stimulating the patient's heart. The control signal may be generated when the modified output signal, the (unmodified) output signal or both signals indicate abnormal activity of the patient's heart, such as ventricular fibrillation or ventricular tachycardia.

It is possible that the output signal is received in each of a sequence of time steps. In other words, the output signal may be sampled at a constant sampling rate. However, the sampling rate may also be varied when the method is carried out, e.g. depending on a significant change in one or more characteristics of the output signal.

Generally, in this case, identifying the undesired pulse pattern may comprise:
- determining an amplitude of the output signal in each time step;
- determining whether the amplitude of each time step is within a predetermined value range;
- when the amplitude is within the predetermined value range for at least a predetermined number of consecutive time steps: recognising that the undesired pulse pattern is present; and/or
- when the amplitude is outside the predetermined value range for at least a predetermined number of consecutive time steps: recognising that the undesired pulse pattern is absent.

For example, the predetermined value range may have been determined from an amplitude of at least one previously identified repetition of the undesired pulse pattern and/or from a trend determined from an amplitude of at least two previously identified repetitions of the undesired pulse pattern (see above).

For example, the predetermined number of consecutive time steps may have been determined depending on the sampling rate.

This allows easy implementation of the method in software and/or hardware.

It should be noted that possible features and advantages of embodiments of the invention are described above and below partly with reference to a method for controlling an implantable medical device and partly with reference to a controller specifically designed for carrying out this method. A person skilled in the art will recognize that the features described for individual embodiments may be suitably transferred to other embodiments in an analogous manner, may be adapted and/or interchanged to arrive at further embodiments of the invention and possibly synergistic effects.

Advantageous embodiments of the invention are further explained below with reference to the accompanying drawings. Neither the drawings nor the description are to be construed as limiting the invention.
Fig. 1 shows a patient carrying an implantable medical device according to an embodiment of the invention.
Fig. 2 shows the implantable medical device in more detail.
Fig. 3 shows a diagram illustrating a sample of an electrical heart signal during abnormal activity of a patient's heart.
Fig. 4 shows a diagram illustrating a sample of an electrical pacing signal generated by a pacemaker.
Fig. 5 shows a diagram illustrating a sample of an output signal generated by a sensor of the implantable medical device during abnormal activity and pacing of the patient's heart.
Fig. 6a to Fig. 6c show possible shapes of an undesired pulse pattern in the output signal.
Fig. 7 shows a diagram illustrating a sample of the output signal processed with a method according to an embodiment of the invention.
Fig. 8 shows a diagram illustrating a sample of the output signal with an undesired pulse pattern similar to a QRS complex.
Fig. 9 shows a diagram illustrating a sample of the output signal with an undesired pulse pattern whose amplitude decreases over time.

The figures are merely schematic and not to scale. Identical reference signs in the figures denote identical features or features having the same effect.

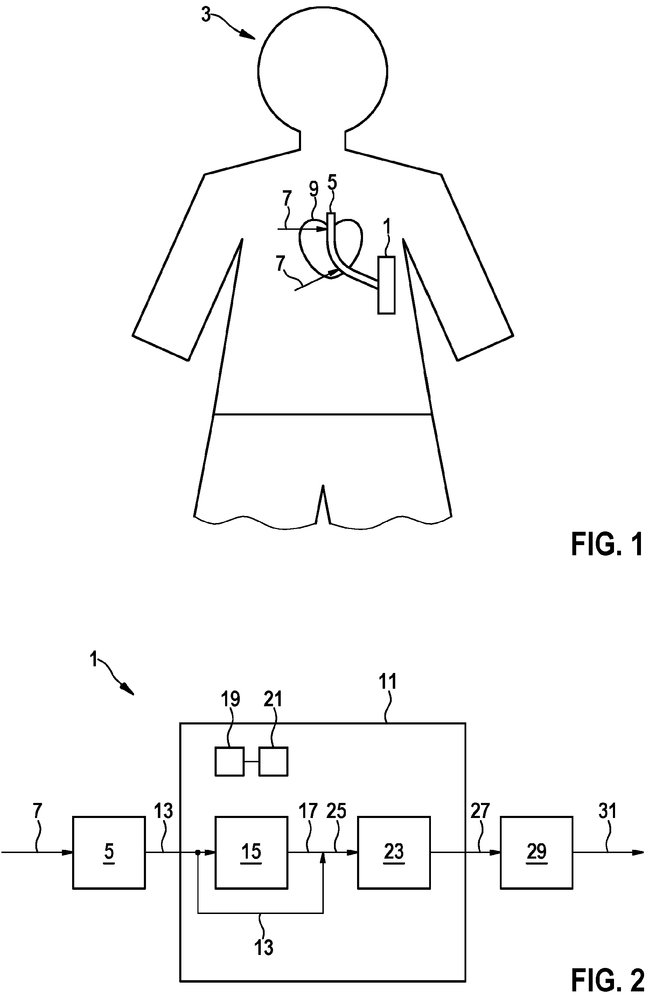

Fig. 1 shows an implantable medical device 1, here a subcutaneous (i.e. extravascular) implantable cardioverter-defibrillator (ICD) 1, implanted in a patient 3.

In this example, the patient 3 further carries a separate pacemaker (not shown), e.g. a leadless pacemaker, which is not operably coupled to the ICD 1.

The ICD 1 comprises a sensor 5 implanted in the patient's body and adapted to sense an electrical heart signal 7 from the patient's heart 9 at different portions of the patient's body.

As shown in Fig. 2, the ICD 1 further comprises a controller 11 for controlling the ICD 1 on the basis of an output signal 13 generated by the sensor 5 from the heart signal 7.

The controller 11 may comprise a signal processing unit 15 configured to generate a modified output signal 17 from the output signal 13 with a method described in more detail below. The method may be carried out by a processor 19 of the controller 11 by executing instructions of a computer program stored in a memory 21 of the controller 11.

The controller 11 may further comprise a control unit 23 configured to generate, from an input signal 25, a control signal 27 for controlling a pulse generator 29 of the ICD 1. The pulse generator 29 may be configured to generate, from the control signal 27, an electrical pulse 31 for electrically stimulating the patient's heart 9.

The signal processing unit 15 may provide the modified output signal 17, the (unmodified, or filtered and/or amplified) output signal 13 or both signals 13, 17 as the input signal 25.

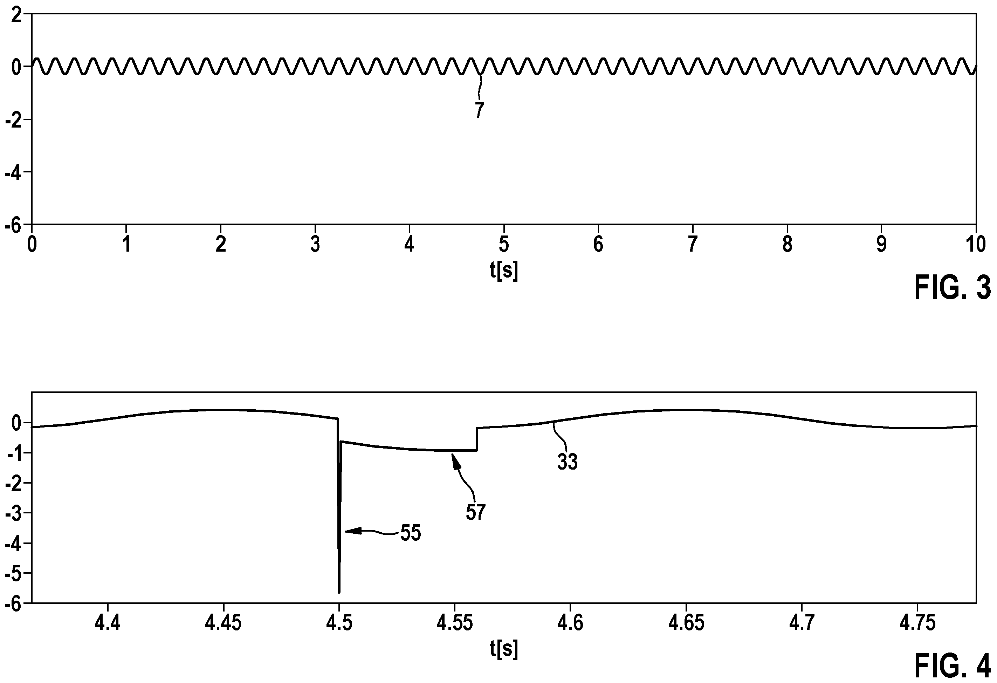

Fig. 3 shows an amplitude of the heart signal 7 during abnormal activity of the patient's heart 9, here during ventricular fibrillation.

Fig. 4 shows an amplitude of an electrical pacing signal 33 generated by the pacemaker for pacing the patient's heart 9.

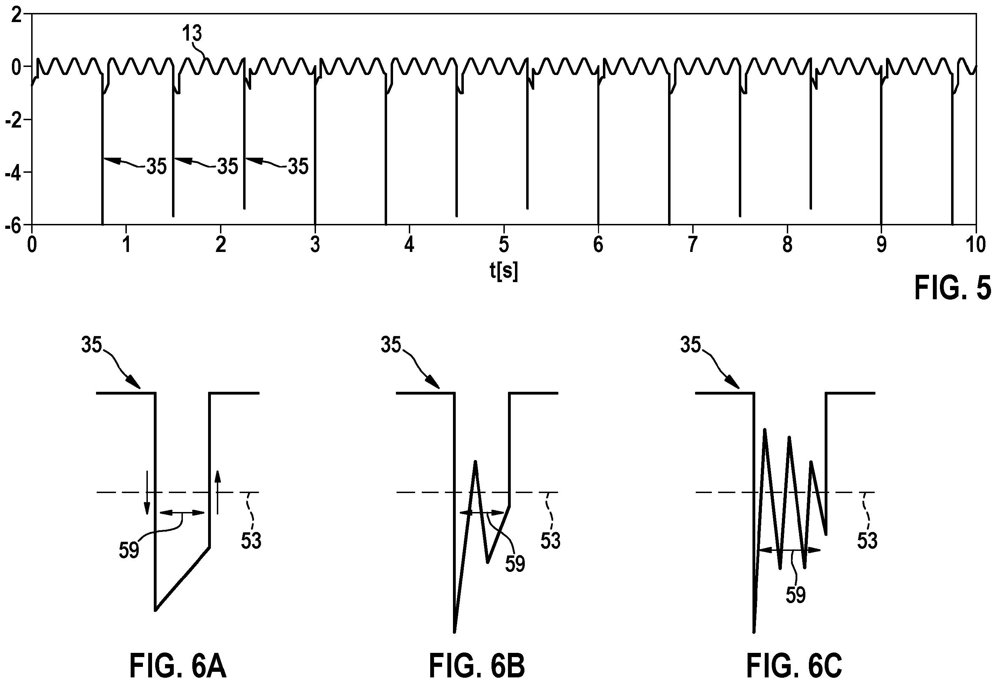

Fig. 5 shows an amplitude of the resulting output signal 13 generated by the sensor 5 when sensing the heart signal 7 during pacing of the patient's heart 9 with the pacing signal 33. As shown here, the output signal 13 may comprise an undesired pulse pattern 35 that repeats at regular intervals. In this example, each repetition of the undesired pulse pattern 35 has the form of a Dirac-like impulse. Such interference in the output signal 13, which may be misinterpreted by the controller 11 as normal activity of the patient's heart 9, can be at least partially eliminated with the following method.

Generally, the method may comprise the following steps.

In a first step, the output signal 13 is received at the signal processing unit 15.

In a second step, the signal processing unit 15 identifies the undesired pulse pattern 35 in the output signal 13.

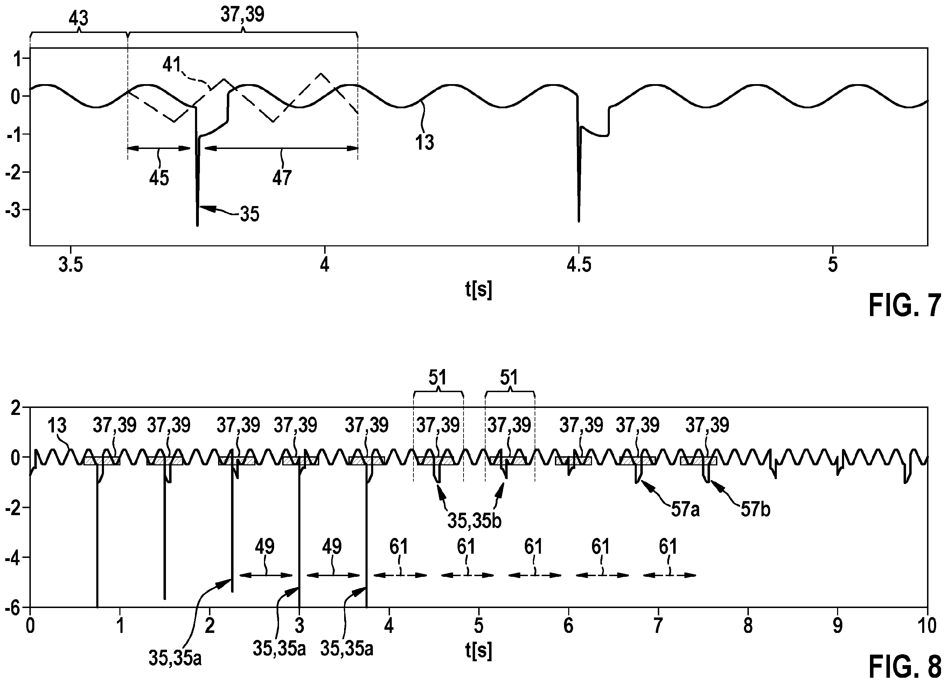

In a third step, the signal processing unit 15 determines a first portion 37 (see Fig. 7) of the output signal 13. The first portion 37 includes the entire undesired pulse pattern 35.

In a fourth step, the signal processing unit 15 generates the modified output signal 17 by replacing the first portion 37 with a predetermined second portion 39 that lacks the undesired pulse pattern 35.

The second portion 39 may include a desired pulse pattern 41 determined by the signal processing unit 15 from a third portion 43 of the output signal 13 outside the undesired pulse pattern 35.

For example, the desired pulse pattern 41 may be determined according to frequencies associated with characteristic peaks found in a spectrum of the third portion 43.

Additionally or alternatively, the desired pulse pattern 41 may be determined according to a degree of periodicity determined from the third portion 43, e.g. using an autocorrelation function.

The desired pulse pattern 41 may also be a mere copy of the third portion 43 (or of a part of it).

As shown in Fig. 7, the first portion 37 may be longer than the undesired pulse pattern 35. More specifically, the undesired pulse pattern 35 may be preceded by a preceding section 45 of a predetermined duration and/or followed by subsequent section 47 of a predetermined duration. The sections 45, 47 may have equal or different durations. In some cases, one or both durations may be set to zero or a negative value.

As shown in Fig. 8, the signal processing unit 15 may be configured to determine a time interval 49 between previously identified consecutive repetitions 35a of the undesired pulse pattern 35 and to estimate, from the time interval(s) 49, one or more time frames 51 in which a current repetition 35b of the undesired pulse pattern 35 is likely to occur.

The signal processing unit 15 may then search for the undesired pulse pattern 35 in the estimated time frame(s) 51.

Optionally, the signal processing unit 15 may determine an actual accuracy with which each repetition 35a, 35b is recognised and compare the accuracy with a required accuracy. When the actual accuracy is below the required accuracy, the signal processing unit 15 may stop searching for the undesired pulse pattern 35 and directly insert the second portion 39 into the estimated time frame(s) 51.

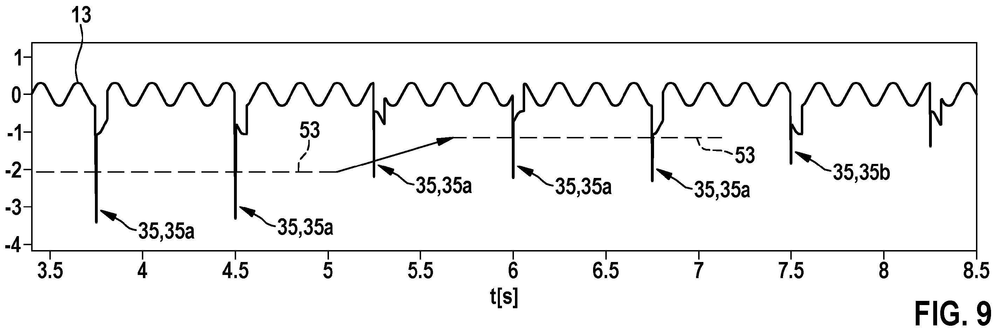

The signal processing unit 15 may optionally determine an amplitude from each previously identified repetition 35a, e.g. a minimum, maximum and/or average amplitude, and use the found amplitude value(s) to determine at least one threshold value 53, as shown in Fig. 9. The signal processing unit 15 may then compare a current amplitude of the output signal 13 with the threshold value(s) 53 to identify a current repetition 35b of the undesired pulse pattern 35.

In particular, the threshold value(s) 53 may be continuously adjusted depending on a significant change in the amplitude of the undesired pulse pattern 35 between two or more previously identified repetitions 35a. In this example, the amplitude decreases in magnitude. As a result, the signal processing unit 15 reduces the magnitude of the threshold value(s) 53 accordingly.

Some of the above figures are described in other words below.

Fig. 4 shows how a signal 33 is perceived that originates from a stimulating device other than the ICD 1, e.g. a pacemaker. For better understanding, the signal 33 is shown here without the heart signal 7 being present at the same time. Such a pacing signal typically includes needle-like pulses 55, with very high amplitude and a pulse width of a few milliseconds, followed by a post-potential 57 due to the remaining charge at the phase boundary of the stimulating electrodes.

Fig. 5 shows a superposition of the two signals 7, 33, as it may appear in the output signal 13 of the ICD 1. It can be seen that pulses 35 with very high amplitude dominate the output signal 13 so that the actual heart signal 7 appears negligible in comparison. Without suitable modification of the output signal 13, the controller 11 could incorrectly assess the rhythm of these pulses 35 as the patient's heart rhythm. From the ICD's point of view, these pulses are to be regarded as disturbances that should be filtered out.

Fig. 6a to Fig. 6c show how an undesired pulse pattern 35 may be detected by means of a threshold value 53.

As shown in Fig. 6a, the undesired pulse pattern 35 may be recognised as present when a time interval 59 between an instant when an absolute value of the amplitude of the output signal 13 exceeds the threshold value 53 (arrow down) and an instant when the absolute value falls below the threshold value 53 again (arrow up) is below a predetermined value.

Fig. 6b and Fig. 6c show examples in which the absolute value falls one or more times below the threshold value 53 within the time interval 59. In this case, the undesired pulse pattern 35 may be recognised as present when the (cumulative) dwell time the absolute value remains below the threshold value 53 is below a predetermined value.

Fig. 8 shows an undesired pulse pattern 35 with a very narrow pulse. As a result, the undesired pulse pattern 35 may not be detected properly in some cases, and different artefacts 57a, 57b of the post-potential 57 may remain in the signal. Such artefacts may look similar to QRS complexes and may therefore cause inaccuracies. In order to eliminate such artefacts, time intervals 49 between previously identified repetitions 35a of the undesired pulse pattern 35 may be determined. This periodicity may then be used to determine estimated time intervals 61 between time frames 51 in which the second portion 39 may be inserted, so that the artefacts 57a, 57b are absent in the modified output signal 17.

It should be noted that, in the claims, the word "comprising" does not exclude other elements or steps, and the indefinite article "a" or "an" does not exclude a plurality. A single processor or controller or other unit may fulfil the functions of several items recited in the claims. The mere fact that certain measures are recited in mutually different dependent claims does not indicate that a combination of these measures cannot be used to advantage. Any reference signs in the claims should not be construed as limiting the scope of the claims.

It will be apparent to those skilled in the art that numerous modifications and variations of the described examples and embodiments are possible in light of the above teaching. The disclosed examples and embodiments are presented for purposes of illustration only. Other alternate embodiments may include some or all of the features disclosed here. Therefore, it is the intent to cover all such modifications and alternate embodiments as may come within the true scope of this invention.

## Claims

1. A computer-implemented method for processing an output signal (13) from a sensor (5) of an implantable medical device (1), in particular an implantable cardioverter-defibrillator (1), the sensor (5) configured to generate the output signal (13) from an electrical heart signal (7) from a patient's (3) heart (9), the method comprising:
receiving the output signal (13);
identifying an undesired pulse pattern (35, 35a, 35b) in the output signal (13);
determining a first portion (37) of the output signal (13), the first portion (37) including the undesired pulse pattern (35, 35a, 35b);
generating a modified output signal (17) by replacing the first portion (37) with a second portion (39) that lacks the undesired pulse pattern (35, 35a, 35b).

2. The method of claim 1,
wherein the output signal (13) has been generated by the sensor (5) during abnormal activity of the patient's (3) heart (9); and/or
wherein the undesired pulse pattern (35, 35a, 35b) is similar to a characteristic pulse pattern occurring in the output signal (13) during normal activity of the patient's (3) heart (9) and/or during pacing of the patient's (3) heart (9) with a pacemaker.

3. The method of one of the previous claims, further comprising:
determining a third portion (43) of the output signal (13), the third portion (43) being outside the undesired pulse pattern (35, 35a, 35b);
determining a desired pulse pattern (41) from the third portion (43);
generating the second portion (39), the second portion (39) including the desired pulse pattern (41).

4. The method of claim 3,
wherein the desired pulse pattern (41) is determined according to frequencies associated with characteristic peaks in a spectrum of the third portion (43); and/or
wherein the desired pulse pattern (41) is determined according to a degree of periodicity determined from the third portion (43); and/or
wherein at least a part of the desired pulse pattern (41) is a copy of at least a part of the third portion (43).

5. The method of one of the previous claims,
wherein the first portion (37) begins a predetermined time before a beginning of the undesired pulse pattern (35, 35a, 35b) and/or ends a predetermined time after an end of the undesired pulse pattern (35, 35a, 35b).

6. The method of one of the previous claims,
wherein a current repetition (35b) of the undesired pulse pattern (35, 35a, 35b) is identified considering at least one previously identified repetition (35a) of the undesired pulse pattern (35, 35a, 35b).

7. The method of claim 6,
wherein a time frame (51) in which the current repetition (35b) is likely to occur is estimated from a time interval (49) between at least two previously identified repetitions (35a);
wherein the current repetition (35b) is identified in the time frame (51).

8. The method of claim 7,
wherein the current repetition (35b) is not identified in the time frame (51) when an actual accuracy with which the undesired pulse pattern (35, 35a, 35b) has been last identified is below a required accuracy; and/or
when the current repetition (35b) is not identified in the time frame (51): using at least a part of the output signal (13) in the time frame (51) as the first portion (37).

9. The method of one of the previous claims,
wherein the undesired pulse pattern (35, 35a, 35b) is identified by comparing an amplitude of the output signal (13) with at least one threshold value (53).

10. The method of claim 9 in combination with one of claims 6 to 8,
wherein an amplitude of each previously identified repetition (35a) is determined;
wherein the at least one threshold value (53) is determined from the amplitude of at least one previously identified repetition (35a) and/or from a trend determined from the amplitude of at least two previously identified repetitions (35a).

11. A controller (11) for an implantable medical device (1), in particular an implantable cardioverter-defibrillator (1), the controller (11) comprising a processor (19) configured to carry out the method of one of the previous claims.

12. An implantable medical device (1), in particular an implantable cardioverter-defibrillator (1), comprising:
a sensor (5) configured to generate an output signal (13) from an electrical heart signal (7) from a patient's (3) heart (9);
the controller (11) of claim 11.

13. A computer program comprising instructions which, when the program is executed by a processor (19), cause the processor (19) to carry out the method of one of claims 1 to 10.

14. A computer-readable medium comprising instructions which, when executed by a processor (19), cause the processor (19) to carry out the method of one of claims 1 to 10.

## Patentansprüche

1. Computerimplementiertes Verfahren zur Verarbeitung eines Ausgangssignals (13) von einem Sensor (5) eines implantierbaren medizinischen Geräts (1), insbesondere eines implantierbaren Kardioverter-Defibrillators (1), wobei der Sensor (5) so konfiguriert ist, dass er das Ausgangssignal (13) aus einem elektrischen Herzsignal (7) vom Herzen (9) eines Patienten (3) erzeugt, wobei das Verfahren umfasst:
Empfangen des Ausgangssignals (13);
Identifizieren eines unerwünschten Pulsmusters (35, 35a, 35b) in dem Ausgangssignal (13);
Bestimmen eines ersten Abschnitts (37) des Ausgangssignals (13), wobei der erste Abschnitt (37) das unerwünschte Pulsmuster (35, 35a, 35b) enthält;
Erzeugen eines modifizierten Ausgangssignals (17) durch Ersetzen des ersten Abschnitts (37) durch einen zweiten Abschnitt (39), dem das unerwünschte Pulsmuster (35, 35a, 35b) fehlt.

2. Verfahren nach Anspruch 1,
wobei das Ausgangssignal (13) vom Sensor (5) während einer abnormalen Aktivität des Herzens (9) des Patienten (3) erzeugt wurde; und/oder
wobei das unerwünschte Pulsmuster (35, 35a, 35b) einem charakteristischen Pulsmuster ähnelt, das im Ausgangssignal (13) während der normalen Aktivität des Herzens (9) des Patienten (3) und/oder während der Stimulation des Herzens (9) des Patienten (3) mit einem Herzschrittmacher auftritt.

3. Verfahren nach einem der vorhergehenden Ansprüche, das ferner umfasst:
Bestimmen eines dritten Abschnitts (43) des Ausgangssignals (13), wobei der dritte Abschnitt (43) außerhalb des unerwünschten Pulsmusters (35, 35a, 35b) liegt;
Bestimmen eines gewünschten Pulsmusters (41) aus dem dritten Abschnitt (43);
Erzeugen des zweiten Abschnitts (39), wobei der zweite Abschnitt (39) das gewünschte Pulsmuster (41) enthält.

4. Verfahren nach Anspruch 3,
wobei das gewünschte Pulsmuster (41) gemäß Frequenzen bestimmt wird, die mit charakteristischen Spitzen in einem Spektrum des dritten Abschnitts (43) verbunden sind; und/oder
wobei das gewünschte Pulsmuster (41) gemäß einem aus dem dritten Abschnitt (43) bestimmten Periodizitätsgrad bestimmt wird; und/oder
wobei mindestens ein Teil des gewünschten Pulsmusters (41) eine Kopie mindestens eines Teils des dritten Abschnitts (43) ist.

5. Verfahren nach einem der vorhergehenden Ansprüche,
wobei der erste Abschnitt (37) zu einem vorbestimmten Zeitpunkt vor Beginn des unerwünschten Pulsmusters (35, 35a, 35b) beginnt und/oder zu einem vorbestimmten Zeitpunkt nach Ende des unerwünschten Pulsmusters (35, 35a, 35b) endet.

6. Verfahren nach einem der vorhergehenden Ansprüche,
wobei eine aktuelle Wiederholung (35b) des unerwünschten Pulsmusters (35, 35a, 35b) unter Berücksichtigung mindestens einer zuvor identifizierten Wiederholung (35a) des unerwünschten Pulsmusters (35, 35a, 35b) identifiziert wird.

7. Verfahren nach Anspruch 6,
wobei ein Zeitrahmen (51), in dem die aktuelle Wiederholung (35b) wahrscheinlich auftritt, aus einem Zeitintervall (49) zwischen mindestens zwei zuvor identifizierten Wiederholungen (35a) geschätzt wird;
wobei die aktuelle Wiederholung (35b) in dem Zeitrahmen (51) identifiziert wird.

8. Verfahren nach Anspruch 7,
wobei die aktuelle Wiederholung (35b) nicht in dem Zeitrahmen (51) identifiziert wird, wenn eine tatsächliche Genauigkeit, mit der das unerwünschte Pulsmuster (35, 35a, 35b) zuletzt identifiziert wurde, unter einer erforderlichen Genauigkeit liegt; und/oder
wenn die aktuelle Wiederholung (35b) nicht im Zeitrahmen (51) identifiziert wird:
Verwenden mindestens eines Teils des Ausgangssignals (13) im Zeitrahmen (51) als ersten Abschnitt (37).

9. Verfahren nach einem der vorhergehenden Ansprüche,
wobei das unerwünschte Pulsmuster (35, 35a, 35b) durch Vergleichen einer Amplitude des Ausgangssignals (13) mit mindestens einem Schwellenwert (53) identifiziert wird.

10. Verfahren nach Anspruch 9 in Kombination mit einem der Ansprüche 6 bis 8,
wobei eine Amplitude jeder zuvor identifizierten Wiederholung (35a) bestimmt wird;
wobei der mindestens eine Schwellenwert (53) aus der Amplitude mindestens einer zuvor identifizierten Wiederholung (35a) und/oder aus einem Trend bestimmt wird, der aus der Amplitude mindestens zweier zuvor identifizierter Wiederholungen (35a) bestimmt wird.

11. Eine Steuereinheit (11) für ein implantierbares medizinisches Gerät (1), insbesondere einen implantierbaren Kardioverter-Defibrillator (1), wobei die Steuereinheit (11) einen Prozessor (19) umfasst, der so konfiguriert ist, dass er das Verfahren eines der vorherigen Ansprüche ausführt.

12. Implantierbares medizinisches Gerät (1), insbesondere implantierbarer Kardioverter-Defibrillator (1), umfassend:
einen Sensor (5), der so konfiguriert ist, dass er ein Ausgangssignal (13) aus einem elektrischen Herzsignal (7) vom Herzen (9) eines Patienten (3) erzeugt;
die Steuereinheit (11) nach Anspruch 11.

13. Ein Computerprogramm, das Befehle umfasst, die, wenn das Programm von einem Prozessor (19) ausgeführt wird, den Prozessor (19) veranlassen, das Verfahren nach einem der Ansprüche 1 bis 10 auszuführen.

14. Ein computerlesbares Medium, das Anweisungen umfasst, die, wenn sie von einem Prozessor (19) ausgeführt werden, den Prozessor (19) veranlassen, das Verfahren nach einem der Ansprüche 1 bis 10 auszuführen.

## Revendications

1. Procédé mis en œuvre par ordinateur pour traiter un signal de sortie (13) d'un capteur (5) d'un dispositif médical implantable (1), en particulier un défibrillateur automatique implantable (1), le capteur (5) étant configuré pour générer le signal de sortie (13) à partir d'un signal cardiaque électrique (7) provenant du cœur (9) d'un patient (3), le procédé comprenant les étapes consistant à :
recevoir le signal de sortie (13) ;
identifier un motif d'impulsion non souhaité (35, 35a, 35b) dans le signal de sortie (13) ;
déterminer une première partie (37) du signal de sortie (13), la première partie (37) incluant le motif d'impulsion non souhaité (35, 35a, 35b) ;
générer un signal de sortie modifié (17) en remplaçant la première partie (37) par une deuxième partie (39) exempte du motif d'impulsion non souhaité (35, 35a, 35b).

2. Procédé selon la revendication 1,
dans lequel le signal de sortie (13) a été généré par le capteur (5) pendant une activité anormale du cœur (9) du patient (3) ; et/ou
dans lequel le motif d'impulsion non souhaité (35, 35a, 35b) est similaire à un motif d'impulsion caractéristique se produisant dans le signal de sortie (13) pendant l'activité normale du cœur (9) du patient (3) et/ou pendant la stimulation du cœur (9) du patient (3) avec un stimulateur cardiaque.

3. Procédé selon l'une des revendications précédentes, comprenant en outre les étapes consistant à :
déterminer une troisième partie (43) du signal de sortie (13), la troisième partie (43) étant en dehors du motif d'impulsion non souhaité (35, 35a, 35b) ;
déterminer un motif d'impulsion souhaité (41) à partir de la troisième partie (43) ;
générer la deuxième partie (39), la deuxième partie (39) incluant le motif d'impulsion souhaité (41).

4. Procédé selon la revendication 3,
dans lequel le motif d'impulsion souhaité (41) est déterminé selon des fréquences associées à des pics caractéristiques dans un spectre de la troisième partie (43) ; et/ou
dans lequel le motif d'impulsion souhaité (41) est déterminé selon un degré de périodicité déterminé à partir de la troisième partie (43) ; et/ou
dans lequel au moins une partie du motif d'impulsion souhaité (41) est une copie d'au moins une partie de la troisième partie (43).

5. Procédé selon l'une des revendications précédentes,
dans lequel la première partie (37) démarre à un temps prédéterminé avant un début du motif d'impulsion non souhaité (35, 35a, 35b) et/ou s'arrête à un temps prédéterminé après une fin du motif d'impulsion non souhaité (35, 35a, 35b).

6. Procédé selon l'une des revendications précédentes,
dans lequel une répétition actuelle (35b) du motif d'impulsion non souhaité (35, 35a, 35b) est identifiée en prenant en compte au moins une répétition précédemment identifiée (35a) du motif d'impulsion non souhaité (35, 35a, 35b).

7. Procédé selon la revendication 6,
dans lequel une plage de temps (51) au cours duquel l'apparition de la répétition actuelle (35b) est probable est estimée à partir d'un intervalle de temps (49) entre au moins deux répétitions (35a) précédemment identifiées ;
dans lequel la répétition actuelle (35b) est identifiée dans la plage de temps (51).

8. Procédé selon la revendication 7,
dans lequel la répétition actuelle (35b) n'est pas identifiée dans la plage de temps (51) lorsqu'une exactitude réelle avec laquelle le motif d'impulsion non souhaité (35, 35a, 35b) a été identifié la dernière fois est inférieure à une exactitude exigée ; et/ou
lorsque la répétition actuelle (35b) n'est pas identifiée dans la plage de temps (51) : utiliser au moins une partie du signal de sortie (13) dans la plage de temps (51) en tant que première partie (37).

9. Procédé selon l'une des revendications précédentes,
dans lequel le motif d'impulsion non souhaité (35, 35a, 35b) est identifié en comparant une amplitude du signal de sortie (13) à au moins une valeur de seuil (53).

10. Procédé selon la revendication 9 en association avec l'une des revendications 6 à 8,
dans lequel une amplitude de chaque répétition précédemment identifiée (35a) est déterminée ;
dans lequel l'au moins une valeur de seuil (53) est déterminée à partir de l'amplitude d'au moins une répétition précédemment identifiée (35a) et/ou à partir d'une tendance déterminée à partir de l'amplitude d'au moins deux répétitions précédemment identifiées (35a).

11. Dispositif de commande (11) pour un dispositif médical implantable (1), en particulier un défibrillateur automatique implantable (1), le dispositif de commande (11) comprenant un processeur (19) configuré pour mettre en œuvre le procédé selon l'une des revendications précédentes.

12. Dispositif médical implantable (1), en particulier un défibrillateur automatique implantable (1), comprenant :
un capteur (5) configuré pour générer un signal de sortie (13) à partir d'un signal cardiaque électrique (7) provenant du cœur (9) d'un patient (3) ;
le dispositif de commande (11) selon la revendication 11.

13. Programme informatique comprenant des instructions qui, lorsque le programme est exécuté par un processeur (19), amènent le processeur (19) à mettre en œuvre le procédé selon l'une des revendications 1 à 10.

14. Support lisible par ordinateur comprenant des instructions qui, lorsqu'elles sont exécutées par un processeur (19), amènent le processeur (19) à mettre en œuvre le procédé selon l'une quelconque des revendications 1 à 10.
